# EUROPÄISCHE PATENTSCHRIFT

(11) **EP 0 956 828 B1**
(45) Veröffentlichungstag und Bekanntmachung des Hinweises auf die Patenterteilung: **10.08.2005**
(21) Anmeldenummer: 99106734.9
(22) Anmeldetag: 03.04.1999
(51) Int. Cl.: A61B 17/70

(54) **Osteosynthesevorrichtung mit Korrekturstab**
Osteosynthesis device with correction rod
Dispositif d'ostéosynthèse avec tige de correction

(30) Priorität: 27.04.1998 DE 19818765
(43) Veröffentlichungstag der Anmeldung: 17.11.1999
(73) Patentinhaber: DePuy Spine Sàrl, 2400 Le Locle (CH)
(72) Erfinder: Halm, Henry, 49143 Bissingen-Wissingen (DE); Schäfer, Bernd, 73035 Göppingen (DE)
(74) Vertreter: Dreiss, Fuhlendorf, Steimle & Becker

(56) Entgegenhaltungen:
- DE-A- 19 512 709
- DE-C- 4 110 002
- DE-C- 4 316 542
- DE-U- 9 403 231
- FR-A- 2 650 173
- US-A- 5 667 508
- US-A- 5 735 850

## Beschreibung

Die Erfindung betrifft eine Osteosynthesevorrichtung mit den Merkmalen des Oberbegriffs des Anspruchs 1.

Aus der DE-C-43 16 542 ist eine Knochenschraube bekannt geworden, mit der ein längsgenuteter Stab verdrehsicher fixiert werden kann. Hierfür weist die Knochenschraube einen Gabelkopf auf, in deren Nut der längsgenutete Stab eingelegt werden kann. Der Nutgrund ist mit Längsnuten versehen, welche eine formschlüssige Verbindung mit dem Stab herstellen. Zur Fixierung des Stabes wird auf den Gabelkopf eine Hutmutter aufgeschraubt und mit dieser der Stab im Nutgrund festgeklemmt. Durch die verdrehsichere Festlegung des Stabes an der Knochenschraube können hohe Korrekturkräfte bzw. Haltekräfte übertragen werden. Jedoch bedarf es für die Übertragung von in Längsrichtung des Stabes gerichteten Kräften zusätzlicher Hilfsmittel bzw. zusätzlicher Stäbe.

Aus der DE-C-26 49 942 ist ein Korrekturimplantat bekannt geworden, bei dem derartige Längskräfte über einen Gewindestab optimal in die Knochenschrauben eingeleitet werden können. Auch hier weist die Knochenschraube einen Gabelkopf auf, in welchen der Gewindestab eingelegt wird.

Die Festlegung des Gewindestabes erfolgt mittels zweier Muttern, die auf dem Gewindestab sitzen und teilweise seitlich in den Gabelkopf eingeschraubt werden. Auf diese Weise wird der Gewindestab an der Knochenschraube fixiert. Als nachteilig hat sich bei dieser bekannten Konstruktion herausgestellt, dass das Befestigen des Gewindestabes am Gabelkopf der Knochenschraube, insbesondere das Eindrehen der Muttern in den Gabelkopf umständlich und zeitraubend ist, da die Muttern ausschließlich über einen Gabelschlüssel verdreht bzw. angezogen werden können und ein Verschieben des Gewindestabes nur dann möglich ist, wenn der Abstand der Mutter zum Gabelkopf ausreichend groß ist.

Aus der FR-A-2 650 173, der DE-C-41 10 002 und der DE-U-94 03 213 sind Vorrichtungen gemäß dem Oberbegriff von Anspruch 1 bekannt, bei denen die Knochenschraube einen Gabelkopf aufweist, in den Gabelkopf ein Gewindestab eingelegt werden kann und der Gewindestab mittels eines Befestigungsmittels an der Schraube fixierbar ist. Der Nutgrund ist mit zum Gewinde des Gewindestabes korrespondierenden Rillen versehen. Zwar kann bei diesen Vorrichtungen der Gewindestab nicht mehr aus dem Gabelkopf herausgezogen werden, jedoch besteht immer noch eine gewisse Gefahr, dass sich der Gewindestab innerhalb des Gabelkopfes dreht.

Der Erfindung liegt daher die Aufgabe zugrunde, eine Vorrichtung der eingangs genannten Art derart so auszugestalten, dass die Fixierung des Gewindestabes am Gabelkopf der Knochenschraube noch sicherer ist.

Diese Aufgabe wird mit einer Osteosynthesevorrichtung gelöst, die die Merkmale des Anspruchs 1 aufweist.

Der wesentliche Vorteil der erfindungsgemäßen Ostesynthesevorrichtung wird darin gesehen, dass der Nutgrund, insbesondere die Profilierung des Nutgrundes Mittel aufweist, die eine Längsverschiebung des Gewindestabes im Gabelkopf verhindern. Außerdem weist der Gabelkopf der Knochenschraube ein Schraubmittel auf, mit welchem der Gewindestab im Nutgrund fixiert wird. Durch Lösen dieses Schraubmittels, was relativ einfach und schnell durchführbar ist, kann diese Fixierung gelöst werden, so dass eine Neukorrektur bzw. Nachjustierung erfolgen kann. Allein durch Lösen des am Gabelkopf befestigbaren Schraubmittels, d.h. des mit dem Gabelkopf verschraubten Schraubmittels, wird der Gewindestab freigegeben, so dass er in eine beliebige Lage verschoben werden kann, und zwar unabhängig davon, welche Lage das Schraubmittel einnimmt, sofern es gelöst ist.

Weitere Vorteile, Merkmale und Einzelheiten der Erfindung ergeben sich aus den Unteransprüchen.

Das Schraubmittel kann als Mutter, insbesondere als Hutmutter ausgebildet sein, die auf ein Außengewinde des Gabelkopfes aufgeschraubt ist. Auf diese Weise wird, wie bei der DE-C-43 16 542, der Gewindestab optimal mit der Knochenschraube verbunden. Außerdem bietet die Mutter den wesentlichen Vorteil, dass sie mit einem einzigen Werkzeug betätigbar ist.

Bei einer anderen Ausführungsform ist das Schraubmittel als Madenschraube ausgebildet und in ein Innengewinde des Gabelkopfes eingeschraubt. Die Madenschraube ist z.B. mit einem Innensechskant versehen und kann mit einem herkömmlichen Sechskantwerkzeug betätigt werden. Die Madenschraube kann mit einer Spitze oder einer Ringschneide versehen sein, welche bei angezogener Madenschraube auf dem Gewindestab aufliegt, sich in diesen eingräbt und diesen am Gabelkopf fixiert.

Die Oberflächenprofilierung weist quer zur Längsachse des Gewindestabes verlaufende Querrillen auf. Diese Querrillen korrespondieren zum Gewinde des Gewindestabes. Dabei weisen erfindungsgemäß die Querrillen eine geringere Tiefe auf als das Gewinde des Gewindestabes. Dies hat den wesentlichen Vorteil, dass beim Anziehen des Schraubmittels die Möglichkeit besteht, dass der Gewindestab im Bereich der Anlage an den Querrillen plastisch oder zumindest elastisch verformt wird und dadurch eine sehr hohe Sicherheit gegen eine axiale Verschiebung erzielt wird. Eine weitere Optimierung kann dadurch erzielt werden, dass die Querrillen eine dem Gewinde des Gewindestabes entsprechende Steigung aufweisen. Insbesondere bei einem Feingewinde wird dadurch eine optimale Anlage des Gewindestabes an den Querrillen gewährleistet.

Im Folgenden wird ein besonders bevorzugtes Ausführungsbeispiel beschrieben, wobei auf die Zeichnung Bezug genommen wird.

In der Zeichnung zeigen:
- Figur 1: eine Seitenansicht einer bevorzugten Ausführungsform der Erfindung;
- Figur 2: eine Draufsicht auf den Gabelkopf der Knochenschraube; und
- Figur 3: den Kopf der Knochenschraube mit eingelegtem Gewindestab.

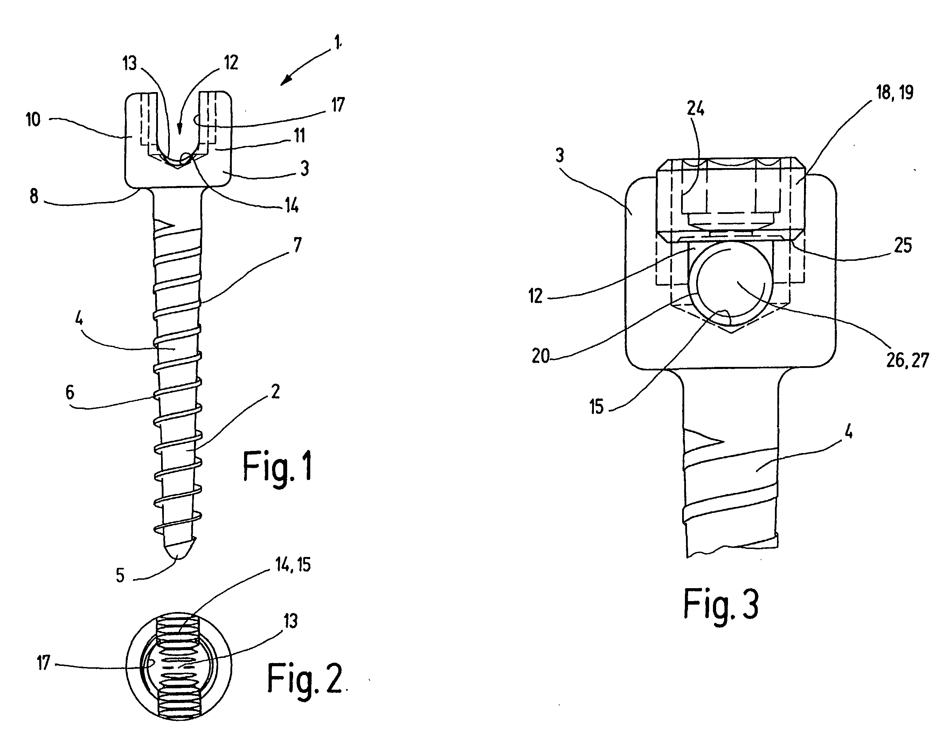

In der Figur 1 ist insgesamt mit 1 eine Knochenschraube bezeichnet, die einen Gewindeschaft 2 und einen Gabelkopf 3 aufweist. Der Gewindeschaft 2 besteht aus einem Schraubenkern 4 mit Schraubenspitze 5 und einem den Schraubenkern 4 umgebenden Gewinde 6. Dabei ist erkennbar, dass der Schraubenkern 4 sich von der Schraubenspitze 5 in Richtung des Gabelkopfes 3 konisch erweitert. Der Durchmesser des Gewindes 6 bleibt jedoch konstant, wohingegen die Breite der Gewindeschneide 7 ausgehend von der Schraubenspitze 5 in Richtung des Gabelkopfes 3 zunimmt. Die Steigung des Gewindes 6 bleibt jedoch über die ganze Länge des Gewindeschaftes 2 konstant. Ferner ist in Figur 1 erkennbar, dass die Unterseite 8 des Gabelkopfes 3 eben, insbesondere kugelförmig ausgebildet ist.

Der Gabelkopf 3 weist zwei Schenkel 10 und 11 auf, zwischen denen eine Nut 12 eingearbeitet ist. Die Nut 12 besitzt sich geringfügig konisch öffnende Nutwände. Der Grund 13 der Nut 12 ist mit einer Oberflächenprofilierung 14 versehen, wie aus Figur 2 ersichtlich ist. Ferner ist erkennbar, dass die Oberflächenprofilierung als Querrillen 15 in den Nutgrund 13 eingearbeitet sind. Die Querrillen 15 werden entweder durch Einfräsen oder durch Einschlagen gebildet.

Das obere Ende des Gabelkopfes 3 ist mit einem Innengewinde 17 versehen, welches sich über etwa die Hälfte der Höhe des Gabelkopfes 3 erstreckt. In dieses Innengewinde 17 ist ein Schraubelement 18 einschraubbar, die als Madenschraube 19 ausgebildet ist.

Die Madenschraube 19 weist an ihrer Oberseite einen Innensechskant 24 auf. An der Unterseite ist die Madenschraube 19 mit einer Ringschneide 25 versehen, die bei angezogener Madenschraube 19 auf einen Korrekturstab 26 drückt. Dieser Korrekturstab 26 (Figur 3) ist als Gewindestab 27 ausgeführt, wobei das Gewinde 20 zu den Querrillen 15 des Nutgrundes 13 korrespondiert.

Wird der Korrekturstab 26, wie in Figur 3 dargestellt, in die Nut 12 des Gabelkopfes 3 eingelegt, dann greift das Gewinde 20 in die Querrillen 15 formschlüssig ein. Anschließend wird die Madenschraube 19 aufgeschraubt, wobei sich die Ringschneide 25 in das benachbarte Gewinde 20 eingräbt. Der Korrekturstab 26 ist auf diese Weise über das in die Querrillen 15 eingreifende Gewinde 20 gegen Verschieben und über die in das Gewinde 20 eingegrabene Ringschneide 25 gegen Verdrehen gesichert. Außerdem werden Drehmomente vom Korrekturstab 26 direkt über die formschlüssige Verbindung mit den Querrillen 15 in den Schraubenkern 4 eingeleitet.

## Patentansprüche

1. Osteosynthesevorrichtung mit einer Knochenschraube (1) mit einem eine Nut (12) aufweisenden Gabelkopf (3) und einem in die Nut (12) des Gabelkopfes (3) einzulegenden Gewindestab (27), wobei der Gewindestab (27) quer zur Längsachse des Gewindestabes verlaufende Querrillen aufweist, der Gabelkopf (3) im Nutgrund (13) eine Oberflächenprofilierung (14) aufweist und der Gabelkopf (3) mit einem den Gewindestab (27) fixierenden Mittel versehen ist, wobei die Oberflächenprofilierung (14) des Nutgrundes (13) die Längsverschiebung des Gewindestab (27) in der Nut (12) verhindernde Querrillen (15) aufweist, und dass das den Gewindestab (27) fixierende Mittel ein Schraubmittel (18) ist, welches am Gabelkopf (3) befestigbar ist, und wobei die Querrillen (15) des Nutgrundes zum Gewinde (20) des Gewindestabes (27) korrespondieren, **dadurch gekennzeichnet, dass** die Querrillen (15) des Nutgrundes eine geringere Tiefe aufweisen als die Querrillen des Gewindes (20) des Gewindestabes (27).

2. Osteosynthesevorrichtung nach Anspruch 1, **dadurch gekennzeichnet, dass** das Schraubmittel (18) eine Mutter, insbesondere eine Hutmutter ist und der Gabelkopf (3) ein Außengewinde aufweist.

3. Osteosynthesevorrichtung nach Anspruch 1, **dadurch gekennzeichnet, dass** das Schraubmittel (18) eine Madenschraube (19) ist und der Gabelkopf (3) ein Innengewinde (17) aufweist.

4. Osteosynthesevorrichtung nach einem der vorhergehenden Ansprüche, **dadurch gekennzeichnet, dass** die Querrillen (15) des Nutgrundes eine dem Gewinde (20) des Gewindestabes (27) entsprechende Steigung aufweisen.

5. Osteosynthesevorrichtung nach einem der vorhergehenden Ansprüche, **dadurch gekennzeichnet, dass** der Gewindestab (27) sandgestrahlt oder beschossen ist.

## Claims

1. An osteosynthesis device, having a bone screw (1) with a bifurcated head (3) that has a groove (12), and having a threaded rod (27) to be placed in the groove (12) of the bifurcated head (3), wherein the threaded rod (27) has transverse grooves extending crosswise to the longitudinal axis of the threaded rod, the bifurcated head (3) at the groove bottom (13) has a surface profiling (14), and the bifurcated head (3) is provided with a means that fixes the threaded rod (27), wherein the surface profiling (14) of the groove bottom (13) has transverse grooves (15) preventing the longitudinal displacement of the threaded rod (27) in the groove (12), and that the means fixing the threaded rod (27) is a screw means (18), which can be secured to the bifurcated head (3), wherein the transverse grooves (15) of the groove bottom correspond to the thread (20) of the threaded rod (27), **characterized in that** the transverse grooves (15) of the groove bottom have a lesser depth than the transverse grooves of the thread (20) of the threaded rod (27).

2. The osteosynthesis device of claim 1, **characterized in that** the screw means (18) has a nut, in particular a cap nut, and the bifurcated head (3) has a male thread.

3. The osteosynthesis device of claim 1, **characterized in that** the screw means (18) is a slug (19), and the bifurcated head (3) has a female thread (17).

4. The osteosynthesis device of one of the preceding claims, **characterized in that** the transverse grooves (15) groove bottom have a pitch corresponding to the thread (20) of the threaded rod (27).

5. The osteosynthesis device of one of the preceding claims, **characterized in that** the correction rod (26) is sandblasted or bombarded.

## Revendications

1. Dispositif d'ostéosynthèse composé d'une vis pour ancrage osseux (1) composée d'une tête fourchue (3) présentant une rainure (12) et d'une tige filetée (27) destinée à être introduite dans la rainure (12) de la tête fourchue (3), la tige filetée (27) présentant des stries transversales à son propre axe longitudinal, la tête fourchue (3) présentant au fond de la rainure (13) une surface profilée (14) et la tête fourchue (3) étant dotée d'un moyen d'immobilisation de la tige filetée (27), la surface profilée (14) du fond de rainure (13) présentant des stries transversales (15) empêchant le déplacement longitudinal de la tige filetée (27) dans la rainure (12), le moyen d'immobilisation de la tige filetée (27) étant un moyen à visser (18) apte à être fixé à la tête fourchue (3) et les stries transversales (15) du fond de rainure correspondant au filetage (20) de la tige filetée (27), **caractérisé en ce que** les stries transversales (15) du fond de rainure ont une profondeur inférieure à celle des stries transversales du filetage (20) de la tige filetée (27).

2. Dispositif d'ostéosynthèse selon la revendication 1, **caractérisé en ce que** le moyen à visser (18) est un écrou, notamment un écrou borgne, et **en ce que** la tête fourchue (3) présente un filetage mâle.

3. Dispositif d'ostéosynthèse selon la revendication 1, **caractérisé en ce que** le moyen à visser (18) est une vis sans tête (19) et **en ce que** la tête fourchue (3) présente un taraudage (17).

4. Dispositif d'ostéosynthèse selon l'une des revendications précédentes, **caractérisé en ce que** les stries transversales (15) du fond de rainure présentent un pas correspondant à celui du filetage (20) de la tige filetée (27).

5. Dispositif d'ostéosynthèse selon l'une des revendications précédentes, **caractérisé en ce que** la tige filetée (27) a été soumise à un sablage ou à un traitement de surface par impact.
